# EUROPÄISCHE PATENTANMELDUNG

(11) **EP 1 676 584 A1**
(43) Veröffentlichungstag der Anmeldung: **05.07.2006**
(21) Anmeldenummer: 05025036.4
(22) Anmeldetag: 16.11.2005
(51) Int. Cl.: A61K 35/58

(54) **Gemische von Schlangengiften zur Krankheitsbekämpfung**

(30) Priorität: 19.11.2004 DE 102004055891
(71) Anmelder: Wiesner, Henning, 81543 München (DE)
(72) Erfinder: Wiesner, Henning, 81543 München (DE)
(74) Vertreter: Grund, Martin

(57) **Zusammenfassung**

Die Erfindung betrifft die Bereitstellung von Kits, die aus mehreren Schlangentoxinen bestehen. Weiterhin werden Verfahren zur Herstellung solcher Kits zur Verfügung gestellt. Darüber hinaus wird die Verwendung von Schlangentoxinen zur Behandlung von Krankheiten, wie Krebs, Autoimmunkrankheiten, Immunschwächekrankheiten, sowie zur Begleittherapie bakterieller und viraler Infektionen, offenbart. Auch werden pharmazeutische Zusammensetzungen zur Verfügung gestellt, die Schlangentoxine enthalten, und zur Behandlung von Krankheiten geeignet sind.

## Beschreibung

### Stand der Technik

Von den 2700 existierenden Schlangenarten zählt etwa ein Fünftel zu den Giftschlangen (1). Die Verwendung der Gifte spezieller Arten hat in der menschlichen Kulturgeschichte lange Tradition, sei es als Jagdgift bei verschiedenen Völkerstämmen in Amerika und Afrika oder als Therapeuticum von Schamanen sowie heutiger Homöopathie oder Medizin. "Giftschlange" ist jedoch kein zoologischer Ordnungsbegriff, sondern charakterisiert die besondere Fähigkeit dieser Reptilien Gift zu produzieren. Zu den Giftschlangen gehören drei Schlangenfamilien. Es sind dies die Familien der Giftnattern (Elapidae), der Vipern (Viperidae) und der Grubenottern (Crotalidae), die sich in ihrer spezifischen Toxinzusammensetzung, Stärke und Wirkung des Toxins selbst noch in den Gattungen und Unterarten unterscheiden (8).

Schlangen verzehren ihre Beute unzerkleinert. Daher muss die Beute vor dem Verzehren getötet werden, um Verletzungen zu vermeiden. Im Gegensatz zu den Riesenschlangen, die ihre Beute erdrosseln, injizieren Giftschlangen ein hochwirksames Toxingemisch, das die Beute in kurzer Zeit tötet. Schlangengifte stellen ein komplexes Gemisch aus Proteinen oder Polypeptiden dar, die toxische und/oder spezielle enzymatische Eigenschaften besitzen (2). Die Giftzusammensetzung kann sich artspezifisch unterscheiden. Einen Einfluss auf die Giftzusammensetzungen haben die genetisch determinierte Variabilität, die geographische Herkunft, Alter, Ernährungsweise und andere Faktoren. Für Giftschlangen haben die Toxine mehrere Funktionen: Die erste Aufgabe der Toxine ist die Tötung der Beute durch Lähmung der Muskulatur oder Herz-Kreislauf-Versagen (7). Die zweite Aufgabe der Toxine ist die Ünterstützung bei der Verdauung der Beute bevor Fäulnisvorgänge im Innern der Beute einsetzen. Schließlich sind die Giftdrüsen modifizierte Speicheldrüsen, und damit Anhangsorgane des Gastrointestinaltraktes. Die massiven Gewebszerstörungen, die nach Schlangenbissen auftreten, sind oft nichts anderes als die Wirkungen dieser im Schlangengift enthaltenen Verdauungssekrete (7).

Natives Schlangengift (Rohgift) besteht nach heutigem Kenntnisstand vornehmlich zu 90% aus Proteinen und Polypeptiden, sowie zu 10% aus Nukleosiden, Spurenelementen, kleinkettigen Peptiden und Zuckern (5).

Grundsätzlich lässt sich der Peptidanteil von Schlangengift in zwei Komponenten aufteilen: Polypeptide ohne enzymatische Eigenschaften, die beispielsweise als Inhibitoren von Enzymen oder Rezeptorblocker wirken, und toxische oder nichttoxische Enzyme, die beispielsweise verdauend wirken oder in die Agglutination eingreifen können.

Zu den Toxinen gehören die Neurotoxine, die Lähmungen verursachen und eine hohe Spezifität für neuronale Strukturen, Membranen, Rezeptoren und Ionenkanäle besitzen. Diese Toxine greifen das periphere Nervensystem an, und hier die Synapsen (5). Man unterteilt sie postsynaptische und präsynaptische Toxine. Erstere blockieren den nikotinischen Acetylcholin-Rezeptor für den Neurotransmitter Acetylcholin, und verhindern somit eine Weiterleitung des Nervenimpulses. Zu den präsynaptischen Toxinen gehört die neurotoxische Phospholipase A₂, die über einen bislang ungeklärten Mechanismus die Freisetzung von Acetylcholin an der präsynaptischen Membran verhindert. Eine Verstärkung der Neurotransmitterausschüttung bewirken Toxine, die an bestimmte Ionenkanäle binden, wie beispielsweise das Dendrotoxin der Mamba. Einen ähnlichen Effekt haben die sogenannten Zyto- oder Kardiotoxine, die bereits in geringer Konzentration Membranen verändern, indem sie in diesen Membranen Kanäle öffnen und erregbare Membranen depolarisieren. Ebenfalls aus Mambagiften sind Cholinesterase-Inhibitoren bekannt.

Die in Schlangengift enthaltenden Enzyme sind überwiegend auf die Hydrolyse von Phosphorsäureester-, Carbonsäureester-, Glykosid- und Peptidbindungen gerichtet. Durch Spaltung von Glykosid-Bindungen wird vor allem das Mukopolysaccharid Hyaluronsäure gespalten, die der Zell-Zell-Adhäsion dient. Somit werden Haut und Gewebe leichter für andere Stoffe durchdringbar. Phosphorsäure-esterspaltende Enzyme hydrolisieren Phosphomono- und diester. Ihr Beitrag zur Toxität von Schlangengift ist unklar. Zu den Carbonsäureester-spaltende Enzymen gehört die schon oben erwähnte Phospholipase A₂, die Phoslipide unter Abspaltung einer Fettsäure hydrolisieren. Sie blockieren nicht nur die Transmitterfreisetzung, sondern zersetzen auch Skelettmuskeln und Erythrozyten. Die aus Schlangengiften bekannten Proteasen und Peptidasen können sowohl denaturierte als auch native Proteine spalten. Diese Enzyme können unter anderem sowohl die Agglutination als auch die Aggregation von Thrombozyten je nach Wirkweise verhindern oder fördern. Nicht an Vergiftungserscheinungen beteiligt sind die L-Aminosäureoxidasen. Diese Enzyme stehen am Ende des Verdauungsprozesses und oxidieren L-Aminosäuren zu Ketosäuren.

Gemäß dieser Toxin-Klassifizierung lassen sich 3 Familien der therapeutisch interessanten Giftschlangen von einander unterscheiden. Es sind dies die Familien der Giftnattern (Elapidae), der Vipern (Viperidae) und der Grubenottern (Crotalidae), die sich in ihrer spezifischen Toxinzusammensetzung, Stärke und Wirkung des Toxins unterscheiden. Gemeinsam ist den Giften allerdings, dass sich in den meisten das Glykosid-spaltende Enzym Hyaluronidase nachweisen lässt.

Schlangengift ist bereits für verschiedene therapeutische Zwecke vorgeschlagen worden: für die Behandlung verschiedener Krebsarten (US 5,565,431, US 5,232,911), neurodegenerativer Krankheiten (EP 0 063 091, US 4,741,902, DE 2 404 416), Fehlfunktionen des Immunsystems (US 4,741,902, EP 1 391 207) sowie von Viruserkrankungen (EP 1 391 207). Auch der Einsatz von Schlangengiften als Schmerzmittel wird erwähnt (US 5,164,196).

In diesen Ansätzen wurden aus Schlangengift aufgereinigte Komponenten, wie Proteine und Peptide (US 5,565,431), aber auch komplettes Schlangengift (EP 0 063 091) eingesetzt. Insbesondere der Einsatz von aus Schlangengift isolierter Phosphoplipase A₂ als Antitumor Mittel (US 5,232,911; Luis A. Costa et al., Anti-cancer Drugs, 1997, vol. 8, Seiten 829-934) oder zur Behandlung von HIV-Infektionen (EP 1 391 207, David Feinhard et al., J. Clin. Invest., 1999 Sep;104(5):611-8) wird in der Literatur erörtert.

Bekannt ist auch der Einsatz einer Kombination von verschiedenen Schlangengiften oder deren Komponenten zur Behandlung neurodegenerativer Krankheiten (DE 2 404 416). In US 5,565,431 wird die antitumorielle Wirkung zweier Peptide beschrieben, die aus Schlangengiften zweier unterschiedlicher Arten isoliert wurden.

Während sich der Wirkmechanismus der Schlangentoxine bei neurodegenerativen Erkrankungen noch auf den Einfluss von Schlangengift auf die neuronalen Synapsen erklären lässt, ist bislang unbekannt auf welchem Wirkmechanismus die Effekte der Toxine auf Tumorzellen beruhen.

Um eine therapeutische Anwendung zu ermöglichen, kann die Toxität des Schlangengifts gemildert werden. Dazu kann man die Schlangengifte detoxifizieren, d.h. die aktiven Komponenten des Toxins modifizieren, z.B. durch Behandlung mit Formaldehyd, UV-Licht, Oxidation, Erwärmung und andere (siehe DE 2 404 416, Seite 8). Ein anderer Weg ist die Isolierung der Komponenten, denen eine therapeutische Wirkung zugeschrieben wird, bei gleichzeitiger Entfernung alle potentiell toxischen Bestandteile (siehe US 5,232,911, zweite Spalte).

All den hier vorgestellten Therapiemethoden ist gemein, dass ein starres Behandlungsschema eingehalten wird. Mit anderen Worten, eine einmal entwickelte Arznei bestehend aus Schlangengiften, ihren Komponenten oder Kombinationen davon wird in seiner Zusammensetzung nach Beginn der Verabreichung nicht mehr verändert. Weiterhin erfolgt immer nur der Einsatz von maximal zwei Schlangentoxinen.

### Zusammenfassung der Erfindung

Diese Erfindung hat die Aufgabe, ein Kit bereitzustellen, der aus mehreren Schlangentoxinen besteht. Eine weitere Aufgabe der Erfindung ist es, Verfahren zur Herstellung solcher Kits zur Verfügung zu stellen. Weitere Aufgaben der Erfindung sind, Verwendungen der von Schlangentoxinen zur Behandlung von Krankheiten, wie Krebs, Autoimmunkrankheiten, Immunschwächekrankheiten, sowie zur Begleittherapie bakterieller und viraler Infektionen, zur Verfügung zu stellen. Eine weitere Aufgabe der Erfindung ist es auch pharmazeutische Zusammensetzungen zur Verfügung zu stellen, die Schlangentoxine enthalten, und zur Behandlung von Krankheiten geeignet sind.

Die Lösung dieser Aufgaben ergibt sich aus den Patentansprüchen, der nachfolgenden Beschreibung und den Abbildungen. Die Erfindung betrifft einen Kit, umfassend mehr als ein Gemisch von Schlangentoxinen, wobei jedes einzelne Gemisch Schlangentoxine von mindestens drei verschiedenen Schlangenarten enthält, wobei jedes Gemisch sich in mindestens einem Schlangentoxin von den jeweils anderen Gemischen unterscheidet.

Weiterhin betrifft die Erfindung ein Verfahren zur Herstellung eines Kits, dadurch gekennzeichnet, dass man zur Herstellung jedes einzelnen Gemisches die Schlangentoxine von mindestens drei Schlangenarten mischt, wobei sich die Gemische untereinander um mindestens ein Schlangentoxin einer Schlangenart unterscheiden. Auch betrifft die Erfindung die Verwendung von Schlangentoxin zur Herstellung eines pharmazeutischen Kits zur Behandlung von Krankheiten, wie Krebs, Autoimmunkrankheiten, Immunschwächekrankheiten und zur Begleittherapie bakterieller und viraler Infektionen. Des weiteren betrifft die Erfindung eine pharmazeutische Zusammensetzung auf der Basis von Schlangentoxine dadurch gekennzeichnet, dass die Zusammensetzung Schlangentoxine von mindestens drei verschiedenen Schlangenarten enthält. Diese pharmazeutische Zusammensetzung kann zur Behandlung von Krankheiten, wie Krebs, Autoimmunkrankheiten, Immunschwächekrankheiten und zur Begleittherapie bakterieller und viraler Infektionen eingesetzt werden.

### Definitionen

Schlangentoxin: auch Schlangengift genannt, ist das über die Giftzähne aus den Giftdrüsen gewonnen Sekret von Giftschlangen
Natives Schlangentoxin: Schlangengift, das keinerlei Modifikation ausgesetzt ist, mit Ausnahme der Dehydrierung über Lyophilisierung.
Raumtemperatur: 15-25 °C.
Currettage: Ausschabung eines Körperhohlorgans, z.B. Blase, Uterus
BCG: Tuberkulose-Schutzimpfung nach Bacille-Calmette-Guérin
Inst: Installation. Verbringen von Medikamenten in eine Körperhöhle oder Hohlorgan
Anflutungszeit: Dauer in Minuten, die ein auf das Großhirn und das übrige ZNS (Zentralnervensystem) wirksames Präparat nach i.m. Gabe bis zur Entfaltung seiner Wirkungsweise benötigt.
Toleranzstadium: Zustand in dem sich ein Wildtier greifen und behandeln lässt, ohne Abwehreaktionen zu zeigen.

### Beschreibung der Erfindung

Die Erfindung offenbart Kits, die Schlangentoxine enthalten, sowie Verfahren zur Herstellung dieser Kits, Verwendungen diese Kits zur Behandlung von Krankheiten, und pharmazeutische Zusammensetzungen, die Schlangentoxine enthalten. All diesen Ausführungsformen der Erfindung ist gemeinsam, dass Gemische von mindestens drei verschiedenen Schlangentoxinen zum Einsatz kommen. Durch die erfindungsgemäßen Kits, Verfahren, Verwendungen und pharmazeutischen Zusammensetzungen ist es überraschenderweise möglich Krankheiten besonders effektiv zu behandeln. Insbesondere konnte gezeigt werden, dass die erfindungsgemäßen Kits, Verfahren, Verwendungen und pharmazeutischen Zusammensetzungen sich besonders zur Behandlung von Krebs, Autoimmunkrankheiten, Immunschwächekrankheiten und zur Begleittherapie bakterieller und viraler Infektionen eignen. Vorzugsweise werden während der Behandlung die Gemische periodisch gegen Gemische mit einer anderen Zusammensetzung an Schlangengiften ausgetauscht. Die Vorteile der Erfindung liegen insbesondere in einer sehr effektiven Therapie, die keinerlei Nebenwirkungen aufweist, da nur geringe Mengen an Schlangentoxinen eingesetzt werden. Die Verwendung geringer Mengen erlaubt auch einen kostengünstigen Einsatz der teuren Rohtoxine. Die Schlangentoxine müssen auch keinerlei Manipulationen ausgesetzt werden, wie Erhitzung, chemischer Modifikation oder Auftrennung in Einzelkomponenten. Dies hat den Vorteil, dass die Schlangentoxine nicht erst in irgendeiner Form aufwendig aufgearbeitet werden müssen, bevor sie eingesetzt werden können. Außerdem wird dadurch eine Reduzierung der Effektivität der Schlangentoxine durch Modifikation des Schlangengiftes vermieden. Es wird vermutet, dass die Schlangentoxine direkt auf die Zielzellen oder/und indirekt über andere Zellen, z.B. Zellen des Immunsystems, einwirken. Der Vorteil der erfindungsgemäßen Kits, Verfahren, Verwendungen und pharmazeutischen Zusammensetzungen liegt darin, dass durch die in den Gemischen enthaltenen mindestens drei verschiedenen Schlangentoxinen eine Beeinflussung durch mindestens drei verschiedene Wirkstoffe erfolgt. Da drei unterschiedliche Wirkstoffe verwendet werden ist eine allmähliches verringertes Ansprechen der Zellen gegenüber den Schlangentoxinen, wie sie bei der Monotherapie auftreten könnte (6), nicht möglich. Dieser Vermeidung des verringerten Ansprechens der Zellen wird insbesondere in der erfindungsgemäßen Ausführungsform Rechnung getragen, bei der verschiedene Gemische an Schlangentoxinen eingesetzt werden.

### Kits aus Schlangentoxinen:

Die Erfindung betrifft einen Kit, umfassend mehr als ein Gemisch von Schlangentoxinen, wobei jedes einzelne Gemisch Schlangentoxine von mindestens drei verschiedenen Schlangenarten enthält, wobei jedes Gemisch sich in mindestens einem Schlangentoxin von den jeweils anderen Gemischen unterscheidet. In einer weiteren Ausführungsform enthalten die Gemische Schlangentoxine von 3, 4, 5, 6, 7, 8, 9 oder 10 verschiedenen Schlangenarten. Vorzugsweise ist dieser Kit dadurch gekennzeichnet, dass er mindestens 5 verschiedene Gemische enthält. In einer besonders bevorzugten Ausführungsform ist der Kit dadurch gekennzeichnet, dass er mindestens 10 verschiedene Gemische enthält. Vorzugsweise enthält der Kit mindestens 5 verschiedene Gemische, wobei das Gemisch Schlangentoxine von den Schlangenarten enthält, wobei die Schlangenarten ausgewählt sind aus der Gruppe *Naja nivea, Dendroaspis angusticeps, Bitis arietans, Bothrops cotiara, Bothrops alternatus, Bothrops jararaca, Bothrops moojeni, Bothrops jararacussu, Agkistrodon bilineatus* und *Crotalus durissus.* Auch sind Ausführungsformen des Kits mit Schlangentoxinen aus anderen Giftschlangenarten möglich.

### Verfahren zur Herstellung von Kits aus Schlangentoxinen

Eine weitere Ausführungsform der Erfindung betrifft ein Verfahren zur Herstellung von einem Kit umfassend mehr als ein Gemisch von Schlangentoxine, wobei jedes einzelne Gemisch Schlangentoxine von mindestens drei verschiedenen Schlangenarten enthält, wobei jedes Gemisch sich in mindestens einem Schlangentoxin von den jeweils anderen Gemischen unterscheidet, dadurch gekennzeichnet, dass man zur Herstellung jedes einzelnen Gemisches die Schlangentoxine von mindestens drei Schlangenarten mischt, wobei sich die Gemische untereinander um mindestens ein Schlangentoxin einer Schlangenart unterscheiden. In einer weiteren Ausführungsform enthalten die Gemische. Schlangentoxine von 3, 4, 5, 6, 7, 8, 9 oder 10 verschiedenen Schlangenarten. In einer bevorzugten Ausführungsform der erfindungsgemäßen Verfahrens wird zur Herstellung der Gemische lyophilisiertes Schlangengift verwendet. Vorzugsweise wird das lyophilisierte Schlangengift in einer 0,9% Kochsalzlösung Lösung aufgenommen bzw. gelöst. Die Verwendung anderer Lösungen, welche die native Struktur der Proteine erhalten, wie isotonische Lösungen, ist ebenfalls möglich. Vorzugsweise wird das gelöste Schlangengifte sterilfiltriert. Zur Sterilfiltration werden Filter oder Filteranlagen eingesetzt, die Sterilfilter mit einer Porengrößen enthalten, die die Sterilität des Filtrates gewährleisten. Vorzugsweise werden die hergestellten Gemische, seien sie gelöst oder als Feststoff, zur Konservierung gekühlt. Eine Kühlung erfolgt vorzugsweise bei einer Temperatur von 4°C. Vorzugsweise werden die hergestellten Gemische zur Konservierung mit handelsüblichen stabilisierenden Faktoren oder Lösungen versetzt. Solche Faktoren können Antioxidantien, wie beispielsweise Benzylalkohol und andere geeignete Antioxidantien sein. In einer bevorzugten Ausführungsform werden die hergestellten Gemische sterilfiltriert. Vorzugsweise bleibt beim erfindungsgemäßen Verfahren die native Struktur der Peptide erhalten. In einer besonders bevorzugten Ausführungsform der Erfindung werden daher Schlangentoxine der Gemische nicht durch Erhitzung, UV-Bestrahlung, Detoxifikation, Abspaltung oder Auftrennung von Einzelkomponenten oder sonst eine Manipulation, die möglicherweise die Polypeptide in ihrer nativen Struktur beeinträchtigen können, behandelt. Vorzugsweise werden gemäß dem erfindungsgemäßen Verfahren zur Herstellung des Gemisches jeweils 500 mg Schlangentoxin in 500 ml Lösungsmittel aufgenommen, um eine Stammlösung zu bilden.
Vorzugsweise werden aus diesen Schlangentoxinen therapeutische Injektionslösungen hergestellt, bei denen das Rohtoxin von den Vertretern der Familie Elapidae mit einer Konzentration von 0,5 bis 5 µg/ml bzw. einer Konzentration von 0,5, 1, 1,5, 2, 2,5, 3, 3,5, 4, 4,5 oder 5 µg/ml vorliegt und von den Vertretern der Familie Viperidae und Crotalidae mit einer Konzentration von 1 bis 20 µg/ml bzw. einer Konzentration von 1, 1,5, 2, 2,5, 3, 3,5, 4, 4,5, 5, 5,5, 6, 6,5, 7, 7,5, 8, 8,5, 9, 9,5, 10, 10,5, 11, 11,5, 12, 12,5, 13, 13,5, 14, 14,5, 15, 15,5, 16, 16,5, 17, 17,5, 18, 18,5, 19, 19,5 oder 20 µg/ml vorliegt.
In einer weiteren Ausführungsform werden aus diesen Schlangentoxinen therapeutische Injektionslösungen hergestellt, bei denen das Rohtoxin von den Vertretern der Familie Elapidae mit einer Konzentration von 1 bis 5 µg/ml bzw. einer Konzentration von 1, 1,5, 2, 2,5, 3, 3,5, 4, 4,5 oder 5 µg/ml vorliegt und von den Vertretern der Familie Viperidae und Crotalidae mit einer Konzentration von 5 bis 15 µg/ml bzw. einer Konzentration von 5, 5,5, 6, 6,5, 7, 7,5, 8, 8,5, 9, 9,5 ,10, 10,5, 11, 11,5, 12, 12,5, 13, 13,5, 14, 14,5 oder 15 µg/ml vorliegt.

In einer besonders bevorzugten Ausführungsform werden aus diesen Schlangentoxinen therapeutische Injektionslösungen hergestellt, bei denen das Rohtoxin von den Vertretern der Familie Elapidae mit einer Konzentration von 1 µg/ml und von den Vertretern der Familie Viperidae und Crotalidae mit einer Konzentration von 10 µg/ml vorliegt. Es wird jeweils ein Volumen einer therapeutischen Lösung mit jeweils einem Volumen von mindestens zwei weiteren aus anderen Schlangentoxinen gebildeten therapeutischen Lösungen gemischt, und diese gemischte Lösung wird 1:1 mit einem Volumen einer 0,9% Kochsalzlösung verdünnt. Diese letztgenannten Lösungen (erfindungsgemäßen Gemische) sind zur direkten Injektion geeignet. Vorzugsweise sind in dem Kit diese erfindungsgemäßen Gemische in Einheiten enthalten, die pro Einheit eine Injektion erlauben. Die Einheiten können die gleiche oder eine voneinander verschiedene Schlangentoxin-Zusammensetzung haben. Vorzugsweise sind die Einheiten in sterilen Gefäßen verpackt. Besonders bevorzugt ist eine Verpackung in sterile Durchstechfläschchen. Eine Injektion der Einheiten erfolgt vorzugsweise intramuskulär oder subkutan.

### Herstellung eines pharmazeutischen Kits mit Schlangentoxinen

Eine weitere Ausführungsform der Erfindung betrifft die Verwendung von Schlangentoxin zur Herstellung eines pharmazeutischen Kits, umfassend mehr als ein Gemisch von Schlangentoxinen, wobei jedes einzelne Gemisch Schlangentoxine von mindestens drei verschiedenen Schlangenarten enthält, wobei jedes Gemisch sich in mindestens einem Schlangentoxin von den jeweils anderen Gemischen unterscheidet, zur Behandlung von Krebs, Autoimmunkrankheiten, Immunschwächekrankheiten oder zur Begleittherapie bakterieller und viraler Infektionen. In einer weiteren Ausführungsform enthalten die Gemische Schlangentoxine von 3, 4, 5, 6, 7, 8, 9 oder 10 verschiedenen Schlangenarten. Vorzugsweise werden die Gemische des Kits periodisch oder kontinuierlich verabreicht. Periodische Verabreichung bedeutet eine Verabreichung der erfindungsgemäßen Gemische in bestimmten Dosen zu bestimmten Zeitpunkten. Es erfolgt somit keine unterbrechungsfreie Verabreichung der Gemische. Dementsprechend müssen die erfindungsgemäßen Gemische in einer Menge dosiert werden, die den Abbau der wirksamen Bestandteile der Gemische im Körper berücksichtigt. Die Zeiträume zwischen den einzelnen Verabreichungen können sieben Tage betragen, in einer anderen Ausführungsform drei Tage, wiederum in einer anderen Ausführungsform einen Tag, in einer bevorzugten Ausführungsform zwei Tage. In einer Ausführungsform erfolgt die Verabreichung drei mal die Woche, in einer bevorzugten Ausführungsform vier mal die Woche. In einer weiteren Ausführungsform erfolgt die Verabreichung drei mal die Woche, wobei zwischen den Verabreichungen für mindestens einen Tag keine Verabreichung erfolgt. Eine kontinuierliche Verabreichung ist eine Verabreichung, bei der für eine konstante Zufuhr der Gemische in den Körper des Patienten gesorgt ist. Somit ist für eine konstante Konzentration der Gemische im Körper des Patienten gesorgt. Dies ermöglicht eine Behandlung mit einer gleich hohen Dosis über längere Zeiträume. Eine periodische Verabreichung der gelösten Gemische erfolgt über Injektion mit Spritzen, vorzugsweise intramuskulär. In einer alternativen Ausführungsform können die gelösten Gemische auch mit einem Depotwirkstoff kombiniert sein, der eine allmähliche Abgabe des Toxins über eine längere Zeit ermöglicht. Solche Depotwirkstoffe können Polyethlenglykol oder andere geeignete Wirkstoffe sein. Die erfindungsgemäßen Gemische werden vorzugsweise mehrmals pro Woche verabreicht. Besonders bevorzugt ist eine Verabreichung der erfindungsgemäßen Gemische dreimal pro Woche. In einer Ausführungsform der Erfindung wird nach einer Woche Behandlung ein anderes Gemisch zur Behandlung verwendet wird. In einer weiteren Ausführungsform wird für eine Woche ein Gemisch verabreicht und frühestens nach vier Wochen, vorzugsweise nach fünf, insbesondere nach zehn Wochen, dieses Gemisch erstmals wieder verabreicht.

### Pharmazeutische Zusammensetzungen auf der Basis von Schlangentoxinen

Die Erfindung betrifft ferner eine pharmazeutische Zusammensetzung auf der Basis von Schlangentoxine dadurch gekennzeichnet, das die Zusammensetzung Schlangentoxine von mindestens drei verschiedenen Schlangenarten enthält. In einer weiteren Ausführungsform enthalten die pharmazeutischen Zusammensetzungen Schlangentoxine von 3, 4, 5, 6, 7, 8, 9 oder 10 verschiedenen Schlangenarten. Vorzugsweise enthält die erfindungsgemäße pharmazeutische Zusammensetzung Schlangentoxine von Schlangenarten, ausgewählt aus der Gruppe *Naja nivea, Dendroaspis angusticeps, Bitis arietans, Bothrops cotiara, Bothrops alternatus, Bothrops jararaca, Bothrops moojeni, Bothrops jararacussu, Agkistrodon bilineatus* und *Crotalus durissus.* Vorzugsweise wird die erfindungsgemäße pharmazeutische Zusammensetzung zur Behandlung von Krebs, Autoimmunkrankheiten, Immunschwächekrankheiten, oder zur Begleittherapie bakterieller und viraler Infektionen verabreicht.

### Herstellung der pharmazeutischen Zusammensetzungen

Das lyophilisierte Rohtoxin in Kristallform der jeweiligen Art wird bei Raumtemperatur ohne Erhitzung, UV-Bestrahlung, Detoxifikation, Abspaltung oder Auftrennung von Einzelkomponenten oder sonst eine Manipulation, die möglicherweise die empfindlichen Polypeptide beeinträchtigen kann, in 0,9% Kochsalzlösung zur Lösung gebracht. Für jede Schlangenart wird eine eigene Stammlösung hergestellt, indem 500 mg Toxin in 500 ml 0,9% Kochsalzlösung gelöst wird. Danach erfolgt eine sterile Filtration (Minisart plus, SM 17823) und sterile Abfüllung in 5 ml Durchstechfläschchen.

Aus dieser Stammlösung wird die Therapeutische Injektionslösung durch Verdünnung mit 0,9% Kochsalzlösung so hergestellt, dass von dem Rohtoxin von den Vertretern der Familie der Elapidae 1 µg/ml und für die Familien der Viperidae und Crotalidae 10 µg/ml enthalten sind. In einer weiteren Ausführungsform kann die Dosis der verabreichten Schlangengiften reduziert sein. Vorzugsweise erfolgt die Reduzierung dabei nach Erreichung eines Therapieerfolgs.

### Verträglichkeitstest bei Mäusen

Die Mischungen der Schlangentoxine werden auf ihre Verträglichkeit in der Maus überprüft. Dafür wird eine intraperitonale Injektion mit den erfindungsgemäßen Gemischen durchgeführt. Verträglichkeit wird durch das Ausbleiben jeglicher krankhafter Symptome angezeigt.

### Verträglichkeitstest beim Menschen

In einer weiteren Ausführungsform werden die Mischungen der Schlangentoxine auf ihre Verträglichkeit im Menschen überprüft. Von den therapeutischen Injektionslösungen werden je 0,05 - 0,1 ml als Quaddel subcutan auf der Innenseite des Unterarms bei 10 verschiedenen Probanden injiziert. Diese Injektion wird reaktionslos vertragen.

### Legenden zu den Abbildungen

- Figur 1:: Sonogramm (mit Sonosite vet 180+ manueller oder Adapter-integrierter 4-2 MHz Convexsonde, alle Untersuchungen wurde mit diesem Instrument durchgeführt) der Vagina eines Indischen Elefanten mit Gebärmutterkrebs (23. Mai 2003). Die Blutgefäße sind geweitet. Hämorrhagien sind erkennbar.
- Figur 2:: Wie in Figur 1, aber aus anderer Perspektive.
- Figur 3:: Sonogramme des Tumors bei Indischem Elefant mit Gebärmutterkrebs nach Beginn (2. Juni 2003) der Behandlung. Neoplastische Gewebe füllte immer noch die Vaginalhöhlung aus. Wegen der Regression des Tumors war eine exakte Messung des Tumor erstmals möglich (12x7 cm). Außerdem hatten sich die zuvor geweiteten Blutgefäße zusammengezogen. Die zuvor ungeordnete kavernöse Struktur des Tumors hatte sich zu einem homogeneren Gewebe hin verändert.
- Figur 4:: Wie in Figur 3, aber aus anderer Perspektive. Die vaginale Mucosa war am 2. Juni 2004 im Vergleich zur Untersuchung am 23. Mai 2003 besser sichtbar.
- Figur 5:: Sonogramme des Tumors bei Indischem Elefant mit Gebärmutterkrebs nach Beginn der Behandlung (26. Januar 2004). Die Vaginalhöhlung war gut definiert erkennbar, obwohl der Tumor den größten Teil des vaginalen Lumens ausfiillte. Erstmals war der Tumor wegen seiner reduzierten Größe im Sonogramm vollständig sichtbar (4,6 cm x 6,5 cm x 16,6 cm, Fig. 3.1). Insgesamt war das neoplastische Gewebe relativ homogen. Die zuvor geweiteten Blutgefäße waren durch Narbengewebe ersetzt. Es wurde kein pathologisches Material (Mucus, Blut, Wundsekrete) in der Vaginalhöhlung entdeckt.
- Figur 6:: Wie in Figur 5, aber aus anderer Perspektive. Zystische Strukturen erkennbar.
- Figur 7:: Sonogramme des Tumors bei Indischem Elefant mit Gebärmutterkrebs nach Beginn der Behandlung (27. April 2004). Die Reduktion der Tumorgröße schreitet voran.
- Figur 8:: Wie in Figur 7, aber anderes Perspektive. Teile des Tumors beginnen sich in Narbengewebe umzuwandeln.
- Figur 9-11:: Wie in Figur 9, aber aus anderer Perspektive.
- Figur 12:: Sonogramme des Tumors bei Indischem Elefant mit Gebärmutterkrebs nach Beginn der Behandlung am 29.9.04: Canalis urogenitalis, Die zuvor entdeckten multiple zystischen Formation waren nicht mehr nachweisbar.
- Figur 13:: Wie in Figur 12, Vagina/Cerivix. Vaginal-Wand und Caudale Offnung klar nachweisbar. Die Größe des Tumors betrug nur noch 4,3 cm x 5,8 cm x 14,3 cm.
- Figur 14:: Wie in Figur 13, aber andere Perspektive. Tumor heftet nicht mehr an der dorsalen Vaginal-Wand an.
- Figur 15:: Wie in Figur 13, aber andere Perspektive. Keine Zyten mehr erkennbar. Im Vergleich zu den vorherigen Untersuchungen erschien das neoplastische Gewebe noch homogener.

### Beispiele

In den folgenden Beispielen wurden Schlangentoxine der folgenden Schlangenarten eingesetzt:

### Familie Elapidae:

(1) Naja angusticeps

### Familie Viperidae:

(2) Bitis ariet

### Familie Crotalidae:

(3) Bothrops cotiara
(4) Bothrops alternatus
(5) Bothrops jararaca
(6) Bothrops moojeni
(7) Bothrops jararacussu
(8) Agkistrodon bilineatus
(10) Crotalus durissus terrificus

Die angegebenen Ziffern dienen in den weiter unten aufgeführten Beispielen zur Identifikation der jeweiligen Schlangentoxine.

Zur Herstellung der Lösungen wurde das lyophilisierte Rohtoxin in Kristallform der jeweiligen Art bei Raumtemperatur ohne Erhitzung, UV-Bestrahlung, Detoxfikation, Abspaltung oder Auftrennung von Einzelkomponenten oder sonst eine Manipulation, die möglicherweise die empfindlichen Polypeptide hätte beeinträchtigen können, in 0,9% Kochsalzlösung zur Lösung gebracht. Für jede Art wurde eine eigene Stammlösung hergestellt, indem 500 mg Toxin in 500 ml 0,9% Kochsalzlösung gelöst wurde. Danach erfolgte eine sterile Filtration (Minisart plus, SM 17823) und sterile Abfüllung in 5 ml Durchstechfläschchen. Lagerung bei 4 °C über 12 Monate möglich.

Aus dieser Stammlösung wurde die therapeutische Injektionslösung durch Verdünnung mit 0,9% Kochsalzlösung so hergestellt, dass von dem Rohtoxin von den Vertretern der Familie der Elapidae 1 µg/ml und für die Familien der Viperidae und Crotalidae 10 µg/ml enthalten waren außer in den Fällen, in denen therapeutische Injektionslösungen mit anderen Konzentrationen eingesetzt wurden. Von den therapeutischen Lösungen der Schlangen 1-10 wurden mindestens je 3 verschiedene therapeutische Lösungen ä 1 ml oder 4 ä 0,5 ml gemischt, nochmals 1:1 mit 0,9% Kochsalzlösung verdünnt und intramuskulär injiziert. Bei der Verwendung von 4 verschiedenen Schlangentoxinen in einem Gemisch wurde die Endkonzentration jedes einzelnen Toxins um 25% gesenkt im Vergleich zu Gemischen mit drei Schlangentoxinen.

### Beispiel 1 (Verabreichung von Schlangentoxinen bei Blasentumor):

M/*1936; Blasentumor: Erstdiagnose: 03/99
Gering diff. Urothel-Ca pT1G3. Nach Curettage insgesamt 9 BCG-Instill. im Zeitraum von 07-10/99; keine Chemotherapie
Zusätzliche Toxintherapie ab 07/99 bis heute (Stand 08/04). Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war.

**Tabelle 1: In den jeweiligen Wochen verwendete Gemische (Verabreichungsschema):**

| Woche | Schlangentoxine |
|---|---|
| 1 | 1,2,3 |
| 2 | 1,2,4 |
| 3 | 1,2,5 |
| 4 | 1,2,6 |
| 5 | 1,2,7 |

Nach Woche 5 wurde das Verabreichungsschema wiederholt.
Rückbildung des Blasentumors bis 12/99 und seit dem kein Rezidiv, Allgemeinzustand, Allgemeinbefinden: ohne Befund.

### Beispiel 2 (Verabreichung von Schlangentoxinen bei Morbus Hodgkin):

W/*1976; Erstdiagnose: 01/02 Morbus Hodgkin
nodulär-sklerosierend (ICD10 : C81.9) HD 12, Stadium III b, Chemotherapie in 8 Zyklen BEACOPP; zusätzliche Toxintherapie ab 02/2002 bis heute.

Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war.

**Tabelle 2: In den jeweiligen Wochen verwendete Gemische (Verabreichungsschema):**

| Woche | Schlangentoxine |
|---|---|
| 1 | 1,2,3 |
| 2 | 1,2,4 |
| 3 | 1,5,4 |
| 4 | 6,5,4 |
| 5 | 6,5,7 |

Nach Woche 5 wurde das Verabreichungsschema wiederholt.

Rückbildung der Tumoren ab 8/2002. Seit 03/2003 keine Tumoren mehr nachweisbar. Seither rezidivfrei (Stand 08/04); Allgemeinzustand, Allgemeinbefinden: ohne Befund.

### Beispiel 3 (Verabreichung von Schlangentoxinen bei Brustkrebs/Mammakarzinom):

W/*1943; Mamma Ca, Erstdiagnose: 1985
OP und Chemotherapie; Nachuntersuchung 05/02: Metastase in li. Lungenmittellappen, Pleurakarzinose, linksseitiger Pleuraerguss, gestörte Atemfunktion, Pleuralpunktion. Toxintherapie seit 05/02 ohne Chemotherapie, da nach Ansicht der behandelnden Ärzte therapierefraktärer, infauster Befund.

Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war.

**Tabelle 3: In den jeweiligen Wochen verwendete Gemische (Verabreichungsschema):**

| Woche | Schlangentoxine |
|---|---|
| 1 | 1,2,3 |
| 2 | 1,2,4 |
| 3 | 1,2,5 |
| 4 | 1,2,6 |
| 5 | 1,2,7 |
| 6 | 1,2,8 |
| 7 | 1,2,9 |
| 8 | 1,2,10 |
| 9 | 1,3,10 |
| 10 | 1,4,10 |

Nach Woche 10 wurde das Verabreichungsschema wiederholt.

Nachuntersuchungen 11/02, 05/03 und 05/04: Pleuraerguss: ohne Befund,; keine Hinweise auf Rezidiv; Allgemeinzustand, Allgemeinbefinden, Atemfunktion: ohne Befund. Toxintherapie wird fortgesetzt. Punktion nicht erforderlich.

### Beispiel 4 (Verabreichung von Schlangentoxinen bei Brustkrebs/Mammakarzinom):

W/*1947; Mamma Ca., bds. mit Metastasen in den axillären Lympfknoten bds.,
Erstdiagnose: 01/03
Chemo in 8 Zyklen; Toxintherapie begleitend ab 01/03, bis heute (Stand 08/04):
Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war.

**Tabelle 4: In den jeweiligen Wochen verwendete Gemische (Verabreichungsschema):**

| Woche | Schlangentoxine |
|---|---|
| 1 | 1,2,3 |
| 2 | 1,2,4 |
| 3 | 1,5,4 |
| 4 | 6,5,4 |
| 5 | 6,5,7 |
| 6 | 6,8,7 |
| 7 | 9,8,7 |
| 8 | 9,8,10 |
| 9 | 9,1,10 |
| 10 | 2,1,10 |

Nach Woche 10 wurde das Verabreichungsschema wiederholt.

Ab 01/2003 Tumor nicht nachweisbar. Allgemeinbefinden, Allgemeinzustand: ohne Befund, bisher kein Rezidiv. Toxintherapie wird fortgesetzt.

### Beispiel 5 (Verabreichung von Schlangentoxinen bei Osteosarkom):

W/*1951; Osteosarkom Schultergelenk li., Erstdiagnose: 01/99
Operative Entfernung ohne Chemo; Nachuntersuchung 03/01: 5 cm große Lungenmestastase. Ab 08/01 erfolgte Schlangentoxintherapie.

Die zur Verabreichung eingesetzten Lösungen bestanden aus vier unterschiedlichen Schlangentoxinlösungen. Die Injektion der Lösung erfolgte dreimal pro Woche, wobei zwischen den Injektionen immer mindestens ein Tag Abstand war.

**Tabelle 5: In den jeweiligen Wochen verwendete Gemische (Verabreichungsschema):**

| Woche | Schlangentoxine |
|---|---|
| 1 | 1,2,3,4 |
| 2 | 1,2,3,5 |
| 3 | 1,2,6,5 |
| 4 | 1,7,6,5 |
| 5 | 8,7,6,5 |

Nach Woche 5 wurde das Verabreichungsschema wiederholt.

Stand 08/04: Lungenmetastase nicht mehr mittels Computertomographie (CT) nachweisbar. Weiterhin unter Toxintherapie. Kein Rezidiv nachweisbar. Allgemeinzustand, Allgemeinbefinden: ohne Befund

### Beispiel 6 (Verabreichung von Schlangentoxinen bei Brustkrebs/Mammakarzinom):

W/*1957; Mamma Ca., Erstdiagnose: 03/03
Klassifikation: pT1c, pNO (0/21), LO; Grading: G1; R-Klassifikation: RO; Operation 03/03; keine Chemo; Toxintherapie seit 04/03 bis heute (Stand 08/04)

Die Injektion der Lösung erfolgte dreimal pro Woche, wobei zwischen den Injektionen immer mindestens ein Tag Abstand war.

**Tabelle 6: In den jeweiligen Wochen verwendete Gemische (Verabreichungsschema):**

| Woche | Schlangentoxine |
|---|---|
| 1 | 1,2,3,4 |
| 2 | 1,2,3,5 |
| 3 | 1,2,6,5 |
| 4 | 1,7,6,5 |
| 5 | 8,7,6,5 |
| 6 | 8,7,6,9 |
| 7 | 8,7,10,9 |
| 8 | 8,1,10,9 |
| 9 | 2,1,10,9 |
| 10 | 2,1,10,3 |

Nach Woche 10 wurde das Verabreichungsschema wiederholt.

Mit Einsetzen der Therapie erfolgte Rückbildung des Tumors. Seitdem kein Hinweis auf Rezidiv, Allgemeinzustand; Allgemeinbefinden: ohne Befund Toxintherapie wird fortgesetzt.

### Beispiel 7 (Verabreichung von Schlangentoxinen bei Brustkrebs/Mammakarzinom):

W/*1941; Duktal invasives Mamma Ca., Erstdiagnose: 01/03
CpT1cMoNo/G2RoLoVo, ER neg., PR neg; HER-2-Neu+) Chemo und percutane Radiatio. Onycholyose. Toxintherapie seit 10/03.

Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war.

**Tabelle 7: In den jeweiligen Wochen verwendete Gemische (Verabreichungsschema):**

| Woche | Schlangentoxine |
|---|---|
| 1 | 1,2,3 |
| 2 | 4,2,3 |
| 3 | 4,5,3 |
| 4 | 4,5,6 |
| 5 | 7,5,6 |
| 6 | 7,8,6 |
| 7 | 7,8,9 |
| 8 | 10,8,9 |
| 9 | 10,1,9 |
| 10 | 10,1,2 |

Nach Woche 10 wurde das Verabreichungsschema wiederholt.

Seit 10/2003 ohne Rezidiv (Allgemeinzustand, Allgemeinbefinden ohne Befund). Toxintherapie wird fortgesetzt.

### Beispiel 8 (Verabreichung von Schlangentoxinen bei metastisiertem Prostatatakrebs):

M/*1924; Prostata-Ca mit multiplen ossären Metastasen, Erstdiagnose: 07/97
Hormontherapie bis 06/03. Ab 06/03 orale Chemo (Multosin 280), die wegen Unverträglichkeit (Innapetenz, Apathie, Somnolenz, Gastritis) 12/03 abgesetzt wird. Toxintherapie seit 11/02. Szintigramm im September 2003 zeigte eine osseäre Metastase im Halswirbel von 0,5 cm Durchmesser.

Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war.

**Tabelle 8: In den jeweiligen Wochen verwendete Gemische (Verabreichungsschema):**

| Woche | Schlangentoxine |
|---|---|
| 1 | 1,2,3 |
| 2 | 1,2,4 |
| 3 | 1,5,4 |
| 4 | 6,5,4 |
| 5 | 6,5,7 |

Nach Woche 5 wurde das Verabreichungsschema wiederholt.

Allgemeinzustand, Allgemeinbefinden ohne Befund. Auf dem Szintigramm vom 18.10.2004 kann die früher manifeste und chemotherapeutisch refraktäre osseäre Metastase in der Halszwirbelsäule nicht mehr dargestellt werden. Toxintherapie wird fortgesetzt.

### Beispiel 9 (Verabreichung von Schlangentoxinen bei Darmkrebs):

M/*1940; oklusives Sigma Ca, Erstdiagnose: 07/04
Not-Operation, Regionale Lymphknoten ohne Befund, keine Metastasen sichtbar, keine Chemotherapie. Toxintherapie seit 7/04.

Die zur Verabreichung eingesetzten Lösungen bestanden aus vier unterschiedlichen Schlangentoxinlösungen. Die Injektion der Lösung erfolgte viermal pro Woche.

**Tabelle 9: In den jeweiligen Wochen verwendete Gemische (Verabreichungsschema):**

| Woche | Schlangentoxine |
|---|---|
| 1 | 1,2,3,4 |
| 2 | 5,2,3,4 |
| 3 | 5,6,3,4 |
| 4 | 5,6,7,4 |
| 5 | 5,6,7,8 |

Nach Woche 5 wurde das Verabreichungsschema wiederholt.

Postoperativ ohne Befund (Allgemeinzustand, Allgemeinbefinden ohne Befund), Gewichtszunahme 4 kg, Stand: 08/04. Toxintherapie wird wegen der häufigen postoperativen Metastasierung fortgesetzt.

### Beispiel 10 (Verabreichung von Schlangentoxine bei Lichen ruber planus erosivus mucosae):

M/* 1981, Lichen ruber planus erosivus mucosae großflächig in der ganzen Mundhöhle, Erstdiagnose: 1993.
Therapierefraktär. Toxintherapie seit 10/03.

Die zur Verabreichung eingesetzten Lösungen bestanden aus drei unterschiedlichen Schlangentoxinlösungen. Die Injektion der Lösung erfolgte viermal pro Woche, wobei zwischen den Injektionen immer mindestens ein Tag Abstand war.

**Tabelle 10: In den jeweiligen Wochen verwendete Gemische (Verabreichungsschema):**

| Woche | Schlangentoxine |
|---|---|
| 1 | 1,2,3 |
| 2 | 1,2,4 |
| 3 | 1,2,5 |
| 4 | 1,2,6 |
| 5 | 1,2,7 |

Nach Woche 5 wurde das Verabreichungsschema wiederholt.
Rückgang der Veränderungen um 80%. Stand: 08/04. Toxintherapie wird fortgesetzt.

### Beispiel 11 (Verabreichung von Schlangentoxine bei grippalem Infekt, subfebrilen Infektionen):

Einsatz bei 6 Patienten mit grippalem Infekt und subfebrilen Infektionen unklarer Genese, (Alter und Geschlecht: w 19, m 35, w 40, w 47, m 53, w 60):
Therapieschema wie in Beispiel 1. Stopp der Verabreichung mit dem Abklingen der Symptome nach 4-5 Tagen.

### Beispiel 12 (Verabreichung von Schlangentoxinen bei vegetativer Dystonie):

Einsatz bei 3 Patienten mit Apathie, Inappetenz und rezidivierenden grippalen Infekten (Alter und Geschlecht: m 40, w 18, m 56):
Therapieschema wie in Beispiel 3. Abklingen der Symptome nach 3-4 Wochen. Danach Stopp der Verabreichung.

### Beispiel 13 (Verabreichung von Schlangentoxinen bei Muskelrheumatismus):

Einsatz bei 3 Patienten mit Muskelrheumatismus (Alter und Geschlecht: m 57, w 52, m 61):
Therapieschema wie in Beispiel 7. Verbesserung der Symptome nach 2-4 Wochen. Danach kontinuierliche Weiterbehandlung mit nur einer Injektion pro Woche. Es wurden hierzu therapeutische Injektionslösungen hergestellt, bei denen das Rohtoxin von den Vertretern der Familie Elapidae mit einer Konzentration von 0,5 bis 5 µg/ml und von den Vertretern der Familie Viperidae und Crotalidae mit einer Konzentration von 1 bis 20 µg/ml vorlag. Zu Beginn der Therapie eingesetzte Konzentrationen an Toxin wurden bis zum Ende der Therapie entweder beibehalten oder allmählich abgesenkt.

### Beispiel 14 (Verabreichung von Schlangentoxinen bei bakteriellen und viralen Infektionen):

Bei Haus-, Zoo- und Wildtieren Einsatz bei bakteriellen und viralen Infektionen zur Beschleunigung der Heilungstendenz (Tabelle 11):

| Tierart | Diagnose | Therapie und Therapieverlauf |
|---|---|---|
| 18 Haushund *(Canis familiaris*) | Virusbedingter Zwingerhusten und Diarrhöe bei Welpen | Zusätzlich zur symptomatischen Therapie Therapieschema wie in Beispiel 10. Deutliche Besserung des Allgemeinbefindens nach einem Tag Therapie. Abkürzung der Heilungszeit um ungefähr 1 Woche statt 14 Tage Krankheitsdauer. |
| 6 Wildhunde *(Lycaon pictus)* | | |
| 6 Markhore *(Capra falconeri)* | Kitze 4te-8te Lebenswoche. Infektion mit Coccidien und coliformen Keimen. Blutigwässrige Diarrhöe | Zusätzlich zur Coccidientherapie Therapieschema wie in Beispiel 10, aber mit täglicher Verabreichung. Sistieren der Diarrhöe nach 2 Tagen statt 5-7 Tagen |
| 8 Steinbockkitze *(Capra ibex ibex*) | | |
| 6 Schneeziegen (*Oreamnos americanus*) | | |

Es wurden hierzu therapeutische Injektionslösungen hergestellt, bei denen das Rohtoxin von den Vertretern der Familie Elapidae mit einer Konzentration von 0,5 bis 5 µg/ml und von den Vertretern der Familie Viperidae und Crotalidae mit einer Konzentration von 1 bis 20 µg/ml vorlag. Zu Beginn der Therapie eingesetzte Konzentrationen an Toxin wurden bis zum Ende der Therapie entweder beibehalten oder allmählich abgesenkt.

### Beispiel 15 (Verabreichung von Schlangentoxinen bei Gebärmutterkrebs eines Elefanten):

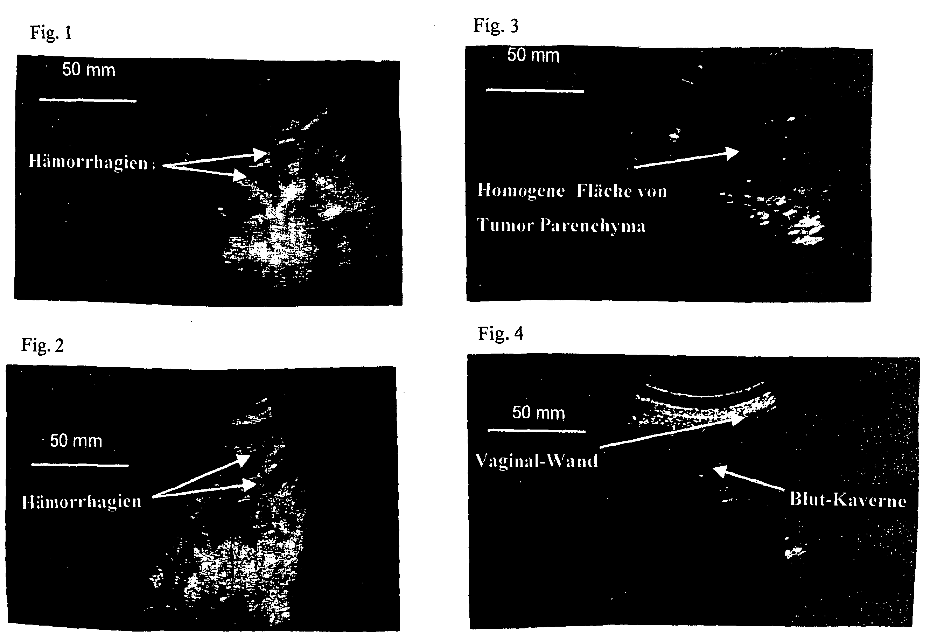

W/*1966, Indischer Elefant, Cervix-Ca., Erstdiagnose: 05/02
Ultraschallbild zeigt einen kavernösen Tumor von Fußballgröße, der nur z.T. mit dem portablen Gerät Sonsonite Vet 180 + darstellbar ist. Messbarer Umfang: 20 cm x 24 cm. Rezidivierende Blutungen aus der Scheide (siehe Figuren 1-2).

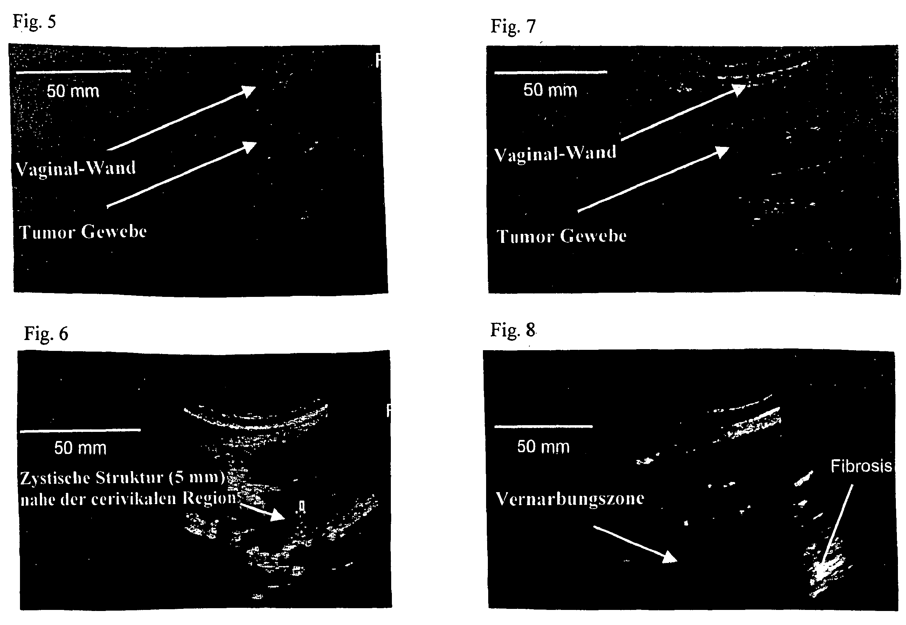

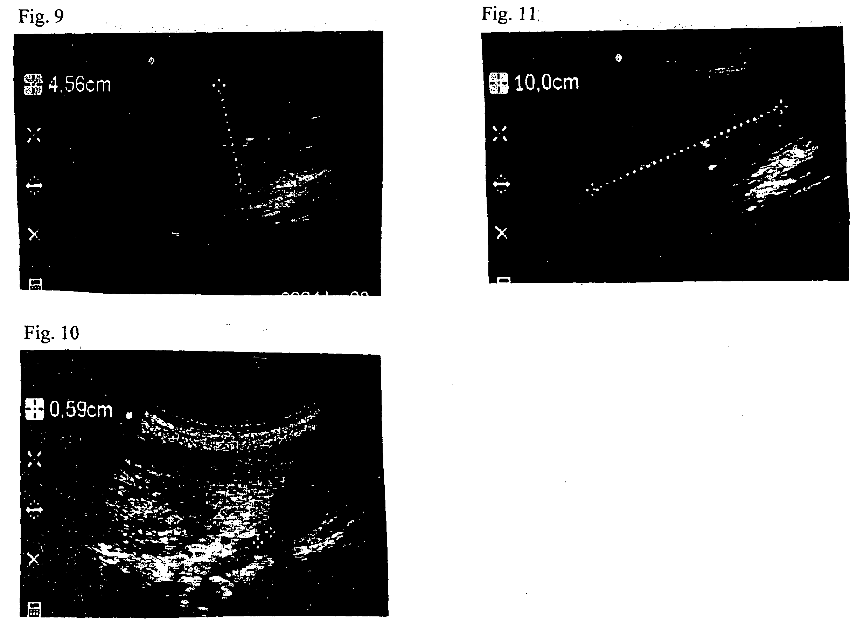

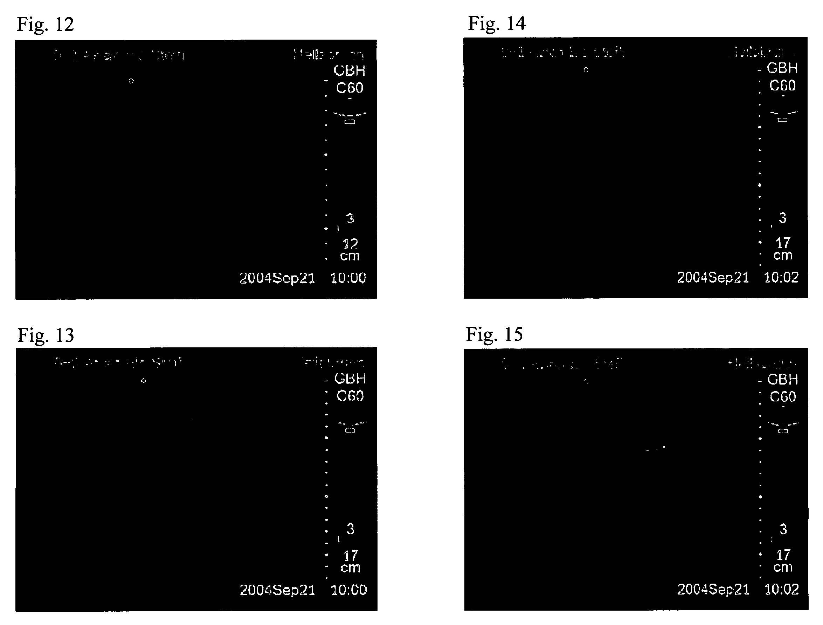

Therapieschema wie in Beispiel 3, aber mit 3 x Humandosis ab 05/02. Mit Therapiebeginn Stillstand der Blutungen.
1. Nachuntersuchung 06/03: 12 cm x 7 cm, keine Kavernen mehr (siehe Figuren 3-4)
2. Nachuntersuchung 01/04: Tumor erstmals sonographisch total erfassbar: 4,6 cm x 6,5 cm x 16,6 cm, Tumorgewebe fibrotisiert. Allgemeinzustand, Allgemeinbefinden: ohne Befund, Toxintherapie wird fortgesetzt (siehe Figuren 5-6).
3. Nachuntersuchung 04/04: Weitere Reduktion der Tumorgröße (siehe Figuren 7-11).
4. Nachuntersuchung 09/04: Weitere Schrumpfung des Tumors auf eine Größe von nunmehr 4,3 cm x 5,8 cm x 14,3 cm. Die Vaginalwand war sonographisch klar detektierbar (siehe Figuren 12-15).

### Beispiel 16 (Verabreichung von Schlangentoxinen bei Blasentumor):

M/* 1944; Blasentumor: Erstdiagnose: 05/02
Gering diff. Urothel-Ca Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war. Bei den therapeutische Injektionslösungen lag das Rohtoxin von den Vertretern der Familie Elapidae mit einer Konzentration von 0,5 µg/ml vor und von den Vertretern der Familie Viperidae und Crotalidae mit einer Konzentration von 1 µg/ml.

Verabreichungsschema wie in Tabelle 1.

Nach Woche 5 wurde das Verabreichungsschema wiederholt.
Rückbildung des Blasentumors bis 04/03 und seit dem kein Rezidiv, Allgemeinzustand, Allgemeinbefinden: ohne Befund.

### Beispiel 17 (Verträglichkeitstest bei Mäusen)

Die Schlangentoxinmischungen aus den obengenannten Beispielen wurden auf ihre Verträglichkeit bei Mäusen getestet. Die Gabe von 0,3 ml der therapeutischen Lösung wurde von jeweils 10 weißen Mäusen (Futtermäusen) intraperitoneal (Gewicht Ø 18,64 g) ohne jegliche klinische Symptome toleriert. Todesfälle traten nicht auf.

### Beispiel 18 (Verträglichkeitstest beim Menschen)

Die Schlangentoxinmischungen aus den obengenannten Beispielen wurden auf ihre Verträglichkeit beim Menschen getestet. Von den therapeutischen Injektionslösungen wurden jeweils 0,05 - 0,1 ml als Quaddel subkutan (s.c.) auf der Innenseite des Unterarms bei 10 verschiedenen Probanden injiziert. Diese Injektion wurde reaktionslos vertragen.

### Beispiel 19 (Verabreichung von Schlangentoxinen bei Morbus Hodgkin):

Erstdiagnose: Morbus Hodgkin.

Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war. In diesem Fall waren die therapeutische Injektionslösungen so hergestellt, dass das Rohtoxin von den Vertretern der Familie Elapidae mit einer Konzentration von 0,5 µg/ml vorlag und von den Vertretern der Familie Viperidae und Crotalidae mit einer Konzentration von 20 µg/ml.

**Tabelle 12: In den jeweiligen Wochen verwendete Gemische (Verabreichungsschema):**

| Woche | Schlangentoxine |
|---|---|
| 1 | 1,2,3 |
| 2 | 1,2,4 |
| 3 | 1,5,4 |
| 4 | 6,5,4 |
| 5 | 6,5,7 |

Nach Woche 5 wurde das Verabreichungsschema wiederholt.

### Beispiel 20 (Verabreichung von Schlangentoxinen bei Brustkrebs/Mammakarzinom):

Duktal invasives Mamma Ca.

Die zur Verabreichung eingesetzten Lösungen bestanden aus drei unterschiedlichen Schlangentoxinlösungen. Die Injektion der Lösung erfolgte dreimal pro Woche, wobei zwischen den Injektionen immer mindestens ein Tag Abstand war. In diesem Fall waren die therapeutische Injektionslösungen so hergestellt, dass das Rohtoxin von den Vertretern der Familie Elapidae mit einer Konzentration von 5 µg/ml vorlag und von den Vertretern der Familie Viperidae und Crotalidae mit einer Konzentration von 1 µg/ml.

**Tabelle 13: In den jeweiligen Wochen verwendete Gemische (Verabreichungsschema):**

| Woche | Schlangentoxine |
|---|---|
| 1 | 1,2,3 |
| 2 | 4,2,3 |
| 3 | 4,5,3 |
| 4 | 4,5,6 |
| 5 | 7,5,6 |
| 6 | 7,8,6 |
| 7 | 7,8,9 |
| 8 | 10,8,9 |
| 9 | 10,1,9 |
| 10 | 10,1,2 |

Nach Woche 10 wurde das Verabreichungsschema wiederholt.

### Beispiel 21 (Verabreichung von Schlangentoxinen bei Darmkrebs):

Oklusives Sigma Ca.

Die zur Verabreichung eingesetzten Lösungen bestanden aus vier unterschiedlichen Schlangentoxinlösungen. Die Injektion der Lösung erfolgte viermal pro Woche. In diesem Fall waren die therapeutische Injektionslösungen so hergestellt, dass das Rohtoxin von den Vertretern der Familie Elapidae mit einer Konzentration von 5 µg/ml vorlag und von den Vertretern der Familie Viperidae und Crotalidae mit einer Konzentration von 20 µg/ml. Zusätzlich wurden die Konzentrationen der therapeutischen Lösungen durch Verdünnung noch um 25% gesenkt, da vier Toxine eingesetzt wurden.

**Tabelle 14: In den jeweiligen Wochen verwendete Gemische (Verabreichungsschema):**

| Woche | Schlangentoxine |
|---|---|
| 1 | 1,2,3,4 |
| 2 | 5,2,3,4 |
| 3 | 5,6,3,4 |
| 4 | 5,6,7,4 |
| 5 | 5,6,7,8 |

Nach Woche 5 wurde das Verabreichungsschema wiederholt.

### Literatur

(1) I. Claus und D. Mebs: Schlangengift-Toxine, Naturwissenschaftl. Rundschau, 44. Jhrg. 1, 1-4, 1994
(2) G.G. Habermehl: Gifttiere und ihre Waffen, Springer Verlag, 1994
(3) P. Kidd: TH 1/TH 2 Balance: The Hypothesis, its Limitations and Implications for Health and Disease. Alt. Med. Rev., Vol 8, 3,k 223-246, 2003
(4) Louis Lewin: Die Pfeilgifte, Gerstenberg, 1984
(5) D. Mebs: Gifttiere, Wiss. Verlagsges. mbH Stuttgart, 1992
(6) S. Modrow und D. Falke: Molekulare Virologie, Spektrum, Akad. Verlag, 1997
(7) F.E. Russel: Snake venom poisoning, Scholium Int. Inc., 1980
(8) N.N.: Die Giftschlangen der Erde, Behring-Werke, Mitt., Marburg/Lahn, 1963
(9) Wiesner, H.: Tierschutzrelevante Neuentwicklungen zur Optimierung der Distanzimmobilisation, Tierärztliche Praxis, 1998, 26(G), 225 ff.

## Patentansprüche

1. Ein Kit, umfassend mehr als ein Gemisch von Schlangentoxine, wobei jedes einzelne Gemisch Schlangentoxine von mindestens drei verschiedenen Schlangenarten enthält, wobei jedes Gemisch sich in mindestens einem Schlangentoxin von den jeweils anderen Gemischen unterscheidet.

2. Der Kit gemäß Anspruch 1, **dadurch gekennzeichnet, dass** er mindestens 5 verschiedene Gemische enthält.

3. Der Kit gemäß Anspruch 1, **dadurch gekennzeichnet, dass** er mindestens 10 verschiedene Gemische enthält.

4. Der Kit gemäß Anspruch 1, **dadurch gekennzeichnet, dass** er mindestens 5 verschiedene Gemische enthält, wobei das Gemisch Schlangentoxine von den Schlangenarten enthält, wobei die Schlangenarten ausgewählt sind aus der Gruppe *Naja nivea, Dendroaspis angusticeps, Bitis arietans, Bothrops cotiara, Bothrops alternatus, Bothrops jararaca, Bothrops moojeni, Bothrops jararacussu, Agkistrodon bilineatus* und *Crotalus durissus.*

5. Verfahren zur Herstellung von einem Kit wie in Anspruch 1-4 beansprucht, **dadurch gekennzeichnet, dass** man zur Herstellung jedes einzelnen Gemisches die Schlangentoxine von mindestens drei Schlangenarten mischt, wobei sich die Gemische untereinander um mindestens ein Schlangentoxin einer Schlangenart unterscheiden.

6. Verfahren gemäß Anspruch 5, wobei zur der Herstellung der Gemische lyophilisiertes Schlangengift verwendet wird.

7. Verfahren gemäß Anspruch 6, wobei zur der Herstellung der Gemische das lyophilisierte Schlangengift in 0,9% Kochsalzlösung aufgenommen wird.

8. Verfahren gemäß Anspruch 7, wobei die gelösten Schlangengifte sterilfiltriert werden.

9. Verfahren gemäß Anspruch 5, wobei die hergestellten Gemische zur Konservierung gekühlt werden.

10. Verfahren gemäß Anspruch 5, wobei die hergestellten Gemische zur Konservierung mit stabilisierenden Faktoren oder Lösungen versetzt wird.

11. Verfahren gemäß Anspruch 5, wobei das hergestellte Gemisch zur Konservierung lyophilisiert werden.

12. Verfahren gemäß Anspruch 5, wobei das hergestellte Gemisch sterilfiltriert werden.

13. Das Verfahren gemäß Anspruch 5, wobei die native Struktur der Peptide erhalten bleibt.

14. Das Verfahren gemäß Anspruch 5, wobei die Schlangentoxine der Gemische nicht durch Erhitzung, UV-Bestrahlung, Detoxifikation, Abspaltung oder Auftrennung von Einzelkomponenten oder sonst eine Manipulation, die möglicherweise die Polypeptide in ihrer nativen Struktur beeinträchtigen können, behandelt werden.

15. Das Verfahren gemäß Anspruch 5, wobei zur Herstellung des Gemisches
a. jeweils 500 mg Schlangentoxin in 500 ml Lösungsmittel aufgenommen werden, um für jedes Schlangentoxin eine Stammlösung zu bilden, woraufhin aus dieser Stammlösung eine therapeutische Lösung so hergestellt wird, dass von dem Schlangentoxin der Familie der Elapidae 0,5 bis 5 µg/ml und für die Familien der Viperidae und Crotalidae 1 bis 20 µg/ml enthalten sind
b. und anschließend jeweils ein Volumen einer therapeutischen Lösung mit jeweils einem Volumen vom mindestens zwei weiteren aus anderen Schlangentoxinen gebildeten therapeutischen Lösungen gemischt werden, und anschließend diese gemischte Lösung 1:1 mit einem Volumen einer 0,9% Kochsalzlösung verdünnt wird.

16. Das Verfahren gemäß Anspruch 5, wobei zur Herstellung des Gemisches
a. jeweils 500 mg Schlangentoxin in 500 ml Lösungsmittel aufgenommen werden, um für jedes Schlangentoxin eine Stammlösung zu bilden, woraufliin aus dieser Stammlösung eine therapeutische Lösung so hergestellt wird, dass von dem Schlangentoxin der Familie der Elapidae 1 bis 5 µg/ml und für die Familien der Viperidae und Crotalidae 5 bis 15 µg/ml enthalten sind
b. und anschließend jeweils ein Volumen einer therapeutischen Lösung mit jeweils einem Volumen vom mindestens zwei weiteren aus anderen Schlangentoxinen gebildeten therapeutischen Lösungen gemischt werden, und anschließend diese gemischte Lösung 1:1 mit einem Volumen einer 0,9% Kochsalzlösung verdünnt wird.

17. Das Verfahren gemäß Anspruch 5, wobei zur Herstellung des Gemisches
a. jeweils 500 mg Schlangentoxin in 500 ml Lösungsmittel aufgenommen werden, um für jedes Schlangentoxin eine Stammlösung zu bilden, woraufhin aus dieser Stammlösung eine therapeutische Lösung so hergestellt wird, dass von dem Schlangentoxin der Familie der Elapidae 1 µg/ml und für die Familien der Viperidae und Crotalidae 10 µg/ml enthalten sind
b. und anschließend jeweils ein Volumen einer therapeutischen Lösung mit jeweils einem Volumen vom mindestens zwei weiteren aus anderen Schlangentoxinen gebildeten therapeutischen Lösungen gemischt werden, und anschließend diese gemischte Lösung 1:1 mit einem Volumen einer 0,9% Kochsalzlösung verdünnt wird.

18. Verwendung von Schlangentoxin zur Herstellung eines pharmazeutischen Kits wie in Anspruch 1-5 zur Behandlung von Krebs.

19. Verwendung von Schlangentoxin zur Herstellung eines pharmazeutischen Kits wie in Anspruch 1-5 zur Behandlung von Autoimmunkrankheiten.

20. Verwendung von Schlangentoxin zur Herstellung eines pharmazeutischen Kits wie in Anspruch 1-5 zur Behandlung von Immunschwächekrankheiten.

21. Verwendung von Schlangentoxin zur Herstellung eines pharmazeutischen Kits wie in Anspruch 1-5 zur Begleittherapie bakterieller und viraler Infektionen.

22. Die Verwendung gemäß Anspruch 18-21, wobei die Gemische des Kits periodisch oder kontinuierlich verabreicht werden.

23. Die Verwendung gemäß Anspruch 18-21, wobei die Gemische des Kits per Injektion verabreicht werden.

24. Die Verwendung gemäß Anspruch 18-21, wobei ein Gemisch mehrmals pro Woche verabreicht wird.

25. Die Verwendung gemäß Anspruch 18-21, wobei ein Gemisch dreimal pro Woche verabreicht wird.

26. Die Verwendung gemäß Anspruch 18-21, wobei jeweils nach einer Woche Behandlung ein anderes Gemisch zur Behandlung verwendet wird.

27. Die Verwendung gemäß Anspruch 18-21, wobei für eine Woche ein Gemisch verabreicht wird und frühestens nach vier Wochen nach der Verabreichung dieses Gemisches erstmals wieder dieses zuvor verabreichte Gemisch verabreicht wird.

28. Eine pharmazeutische Zusammensetzung auf der Basis von Schlangentoxine **dadurch gekennzeichnet**, das die Zusammensetzung Schlangentoxine von mindestens drei verschiedenen Schlangenarten enthält.

29. Die pharmazeutische Zusammensetzung von Anspruch 28, wobei die Zusammensetzung Schlangentoxine von 3, 4, 5, 6, 7, 8, 9 oder 10 verschiedenen Schlangenarten enthält.

30. Die pharmazeutische Zusammensetzung von Anspruch 28 oder 29, wobei die Zusammensetzung Schlangentoxine von Schlangenarten enthält, ausgewählt aus der Gruppe *Naja nivea, Dendroaspis angusticeps, Bitis arietans, Bothrops cotiara, Bothrops alternatus, Bothrops jararaca, Bothrops moojeni, Bothrops jararacussu, Agkistrodon bilineatus* und *Crotalus durissus.*

31. Die pharmazeutische Zusammensetzung von Anspruch 28 oder 29 zur Behandlung von Krebs.

32. Die pharmazeutische Zusammensetzung von Anspruch 28 oder 29 zur Behandlung von Autoimmunkrankheiten.

33. Die pharmazeutische Zusammensetzung von Anspruch 28 oder 29 zur Behandlung von Immunschwächekrankheiten.

34. Die pharmazeutische Zusammensetzung von Anspruch 28 oder 29 zur Begleittherapie bakterieller und viraler Infektionen.
